# EUROPEAN PATENT APPLICATION

(11) **EP 2 385 373 A1**
(43) Date of publication of application: **09.11.2011**
(21) Application number: 11172383.9
(22) Date of filing: 01.07.2011
(51) Int. Cl.: G01N 33/68

(54) **Natriuretic peptides in pregnancy**

(71) Applicant: F. Hoffmann-La Roche AG, 4070 Basel (CH); Roche Diagnostics GmbH, 68305 Mannheim (DE)
(72) Inventor: Hess, Georg, 55130 Mainz (DE); Horsch, Andrea, 68259 Mannheim (DE); Zdunek, Dietmar, 82327 Tutzing (DE)
(74) Representative: Dick, Alexander

(57) **Abstract**

The present invention relates to the field of diagnostic measures. Specifically, it relates to a method for assessing cardiac adaptation to pregnancy in a pregnant subject being in any one of week 1 to 22 of gestation. The method is based on the determination of the amount of a brain natriuretic peptide in a sample of said subject, and on the comparison of the, thus, determined amount to a reference amount. Thereby, cardiac adaptation can be assessed. Further encompassed is a method for assessing in a pregnant woman whether fluid retention is the cause of abnormal weight gain during pregnancy. Said method is also based on the determination of the amount of a brain natriuretic peptide and on the comparison of the amount of said brain natriuretic peptide to a reference amount. Further encompassed are a diagnostic device and a kit for carrying out the aforementioned methods.

## Description

The present invention relates to the field of diagnostic measures. Specifically, it relates to a method for assessing cardiac adaptation in a pregnant subject being in any one of week 1 to 22 of gestation. The method is based on the determination of the amount of a brain natriuretic peptide in a sample of said subject, and on the comparison of the, thus, determined amount to a reference amount.. Thereby, cardiac adaptation can be assessed. Further encompassed is a method for assessing in a pregnant woman whether fluid retention is the cause of abnormal weight gain during pregnancy. Said method is also based on the determination of the amount of a brain natriuretic peptide and on the comparison of the amount of said brain natriuretic peptide to a reference amount. Further encompassed are a diagnostic device and a kit for carrying out the aforementioned methods.

Pregnancy may be complicated by pre-existing disorders such as cardiac diseases including systemic hypertension, pulmonary disorders, diabetes mellitus, intestinal diseases, cerebral disorders and many more. In addition diseases may develop during pregnancy such as gestational diabetes mellitus or most importantly preeclampsia. Therefore, mortality and morbidity due to cardiovascular disease is increasing in pregnancy. The physiologic changes during pregnancy serve as stress test on the cardiovascular system. Pregnant women who do not have significant pre-existing disorders or who run a clinically uneventful course of pregnancy are considered to be clinically healthy during the course of pregnancy.

It has however been recognised that pregnancy involved substantial physiological changes specifically related to the heart and the kidney. Such physiological changes occur primarily early in pregnancy. Endocrine changes result in reduced peripheral vascular resistance (Hermsteiner M et al, Gynecol Reprod Biol 2002: 102: 148 - 154). This results in significantly increased uterine blood flow, and is also associated with an increase in blood volume (associated with reduced hematocrit), increased cardiac output as well as heart rate. The increase in maternal blood flow volume starts in the first weeks of pregnancy and has its peak in the late second trimester (Weissgerber T.L. and Wolfe L.A., Appl Physiol Nutr Metab. 2006: 31: 1- 11). As a result of the volume expansion, there is an increase in the compliance of the maternal cardiovascular system resulting in mild increases in maternal left ventricular end-dystolic and end-systolic volumes (Newstead-Angel et al., Expert Rev. Cardiovasc. Ther. 7(12), 2009).

The physiological changes in pregnancy described above are associated with increased cardiac workload and result in cardiac hypertrophy with enlarged myocytes and thus represent a physiological adaptation to the heart. (Kehat I et al., Circulation 2010, 122: 2727 - 2735). Similar adaptations of the heart occur after exercise (Kehat et al), and it has been learned that these adaptations are dependent on the type of exercise (acute and exhaustive exercise vs moderate endurance training) and pre-existing cardiac conditions (Kojda G. et al., Cardiovascular Research 2005: 67: 187 - 197).

Currently there are no easy methods to capture cardiac adaptation (normal or abnormal) primarily early in pregnancy although this has impact on possible complications or restrictions in pregnancy such as exercise.

Another aspect is the diagnosis of a weight increase beyond normal. In general a weight increase within the first trimenon does not occur, during the second trimenon a weekly weight increase of 300 - 400 g is within normal, in the last trimenon a weekly weight increase of 400 to 500 g is within normal. Specifically rapid increase in body weight might be due to water retention associated with edema or have other causes such as increased energy comsumption. A marker which would allow for assessing the underlying cause of weight increased during pregnancy would be highly desirable.

The use of biomarkers for diagnostic purposes is known as such. The brain natriuretic peptide (BNP) belongs to the family of natriuretic hormones. In vivo, BNP is generated by proteolytic cleavage of precursor molecules, resulting in the active hormone (BNP) and an N-terminal fragment (NT-proBNP).

Natriuretic hormone assays have been discussed as diagnostic and prognostic markers in cardiovascular disease (Clerico, A., Emdin, M. (2004). Diagnostic Accuracy and Prognostic Relevance of the Measurement of Cardiac Natriuretic Peptides: A Review. Clinical Chemistry 50:1, 33-50). However, only limited information is available on the use of natriuretic peptides as markers during pregnancy.

The present invention relates to a method for assessing cardiac adaptation to pregnancy in a pregnant subject being in any one of week 1 to 22 of gestation, said method comprising the steps of
a. determining the amount of a brain natriuretic peptide in a sample from said pregnant subject, and
b. comparing the, thus, determined amount of said brain natriuretic peptide to a reference amount, said reference amount being derived from a pregnant subject being in the same stage of gestation,
whereby cardiac adaptation to pregnancy in said subject is to be assessed.

Preferably, cardiac adaptation to pregnancy is assessed, by carrying out the further step of c) assessing cardiac adaptation to pregnancy based on the result of the comparison carried out in step b).

The method of the present invention, preferably, is an ex vivo method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison and/or diagnosis based on said comparison in step (b). More preferably, the method is carried out entirely in an automated manner. In such a case, the diagnostic result which is established in step b) is generated in a suitable output format so that it can be used as an aid for establishing the final clinical diagnosis by, e.g., a medical practitioner.

The term "assessing" as used herein means determining whether the cardiovascular system of a subject as referred to in accordance with the method of the present invention adapts to pregnancy or not. As will be understood by those skilled in the art, such an assessment is usually not intended to be correct for 100% of the subjects for which the assessement is carried out. The term, however, requires that the assessment is correct for a statistically significant portion of the subjects (e.g. a cohort in a cohort study). Thus, the method of the present invention, however, at least provides an aid for establishing a final clinical diagnosis. Whether a portion is statistically significant, can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "subject" as used in accordance with the aforementioned method relates to animals, preferably mammals, and, more preferably, humans. The subject referred to herein shall be pregnant subject and, thus, shall be female. In accordance with the aforementioned method of the present invention, the subject, preferably, shall be in any one of week 1 to 22 of gestation. More preferably, the subject shall be in any one of week 1 to 17 of gestation. Even more preferably, the subject shall be in any one of week 8 to 17 of gestation. Most preferably, the subject shall be in any one of week 14 to 17 of gestation. The duration of pregnancy is, preferably, calculated from the date of conception.

The subject, preferably, does not exhibit impaired renal function. How to assess whether a subject exhibits impaired renal function is well known in the art. Particularly, renal function can be assessed by means of the glomerular filtration rate (GFR). For example, the GFR may be calculated by the Cockgroft-Gault or the MDRD formula (Levey 1999, Annals of Internal Medicine, 461-470). GFR is the volume of fluid filtered from the renal glomerular capillaries into the Bowman's capsule per unit time. Clinically, this is often used to determine renal function. The GFR was originally estimated (the GFR can never be determined, all calculations derived from formulas such as the Cockgroft Gault formula of the MDRD formula deliver only estimates and not the "real" GFR) by injecting inulin into the plasma. Since inulin is not reabsorbed by the kidney after glomerular filtration, its rate of excretion is directly proportional to the rate of filtration of water and solutes across the glomerular filter. In clinical practice however, creatinine clearance is used to measure GFR. Creatinine is an endogenous molecule, synthesized in the body, which is freely filtered by the glomerulus (but also secreted by the renal tubules in very small amounts). Creatinine clearance (CrCl) is therefore a close approximation of the GFR. The GFR is typically recorded in milliliters per minute (mL/min). The normal range of GFR for males is 97 to 137 mL/min, the normal range of GFR for females is 88 to 128 ml/min. Thus, it is particularly contemplated that the GFR of the subject as referred to herein is within this range. Moreover, the subject as referred to in the context of the method present invention, preferably, has a blood creatinine level (in particular a serum creatinine level) of lower than 0.9 mg/dl, more preferably of lower than 1.1 mg/dl and most preferably of lower than 1.3 mg/dl.

Preferably, the subject is apparently healthy with respect to a cardiovascular disease, in particular before pregnancy and/or at the time at which the assessment according to the present invention is carried out. It is particularly envisaged that a subject who is apparently healthy with respect to a cardiovascular disease exhibits a cardiovascular disease which is asymptomatic and/or clinically not apparent. Thus, the cardiovascular disease preferably, shall be an unrecognized disease. Clinical not apparent and/or asymptomatic cardiovascular diseases include inherited or acquired cardiovascular disorders. Preferred inherited cardiovascular disorders are valvular diseases of the aortic, the mitral, the tricuspidalis and the pulomonalis valve, more preferred inherited diseases are ventricular and most preferably atrial septum defects. Acquired cardiovascular disorders include, preferably, valvular disorders (as described for inherited disorders), myocarditis, pericarditis, endocarditis and more preferably cardiovascular disorders. Cardiovascular disorders may be diagnosed by imaging (e.g. echocardiography) as systolic or more preferably as diastolic dysfunction.

In the context of the aforementioned method of the present invention cardiac adaptation to pregnancy shall be assessed. The term "cardiac adaptation to pregnancy" is well known in the art (see also Weiss et al., European Heart Journal (2002) 21, 104 to 115). The term is frequently also referred to as "cardiovascular adaptation". When assessing cardiac adaptation to pregnancy, it shall be assessed whether the cardiovascular system of a subject has adapted, in particular sufficiently adapted, to pregnancy or not. In particular, it shall be assessed whether the cardiovascular system has adequately and/or physiologically adapted to pregnancy or not.

Preferably, the cardiovascular system of a subject has adapted to pregnancy if it is capable of responding to the demands of pregnancy (as it is observed in subjects without cardiovascular disease). Preferably, the cardiovascular system of a subject has not adapted to pregnancy if it is not capable of responding to the demands of pregnancy. Preferably, the cardiovascular system is capable of responding to the demands of pregnancy, if the increase in blood volume and cardiac output is sufficient in order to fulfill the needs of the mother and growing fetus. Preferably, the cardiovascular system is not capable of responding to the demands of pregnancy, if the increase in blood volume and cardiac output is not sufficient in order to fulfill the needs of the mother and growing fetus. Also preferably, the cardiovascular system of a subject has not adapted to pregnancy, if the subject suffers from diastolic dysfunction and/or increased heart wall tension. Moreover, a subject whose cardiovascular system has not adapted to pregnancy is at increased risk of mortality.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma, urine or serum and, most preferably, blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein. More preferably, the sample is derived from a subject being in any one of week of gestation 1 to 22. Even more preferably, the sample is derived from a subject being in any one of week of gestation 1 to 17. Even more preferably, the sample is derived from a subject being in any one of week 8 to 17 of gestation. Most preferably, the sample is derived from a subject being in any one of week 14 to 17 of gestation.

The term "brain natriuretic peptide" comprises Brain Natriuretic Peptide (BNP)-type peptides and variants thereof having the same diagnostic potential (see e.g. Bonow, 1996, Circulation 93: 1946-1950). BNP-type peptides comprise pre-proBNP, proBNP, NT-proBNP, and BNP. The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP). Preferred brain natriuretic peptides according to the present invention are NT-proBNP, BNP, and variants thereof. BNP is the active hormones and has a shorter half-life than their respective inactive counterpart, NT-proBNP. BNP is metabolised in the blood, whereas NT-proBNP circulates in the blood as an intact molecule and as such is eliminated renally. The in-vivo half-life of NTproBNP is 120 min longer than that of BNP, which is 20 min (Smith 2000, J Endocrinol. 167: 239-46.). Preanalytics are more robust with NT-proBNP allowing easy transportation of the sample to a central laboratory (Mueller 2004, Clin Chem Lab Med 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller loc.cit.; Wu 2004, Clin Chem 50: 867-73.). Therefore, depending on the time-course or properties of interest, either measurement of the active or the inactive forms of the brain natriuretic peptide can be advantageous. The most preferred brain natriuretic peptide is NT-proBNP. As briefly discussed above, the human NT-proBNP, as referred to in accordance with the present invention, is a polypeptide comprising, preferably, 76 amino acids in length corresponding to the N-terminal portion of the human NT-proBNP molecule. The structure of the human BNP and NT-proBNP has been described already in detail in the prior art, e.g., WO 02/089657, WO 02/083913 or Bonow loc. cit. Preferably, human NT-proBNP as used herein is human NT-proBNP as disclosed in EP 0 648 228 B1. These prior art documents are herewith incorporated by reference with respect to the specific sequences of NT-proBNP and variants thereof disclosed therein. The NT-proBNP referred to in accordance with the present invention further encompasses allelic and other variants of said specific sequence for human NT-proBNP discussed above. Specifically, envisaged are variant polypeptides which are on the amino acid level at least 60 % identical, more preferably at least 70 %, at least 80 %, at least 90 %, at least 95 %, at least 98% or at least 99 % identical, to human NT-proBNP, preferably, over the entire length. The degree of identity between two amino acid sequences, in principle, can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith 1981, Add. APL. Math. 2:482, by the homology alignment algorithm of Needleman 1970, J. Mol. Biol. 48:443, by the search for similarity method of Pearson 1988, Proc. Natl. Acad Sci. (USA) 85: 2444, by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of human NT-proBNP as long as the said polypeptides have NT-proBNP properties. NT-proBNP properties as referred to herein are immunological and/or biological properties. Preferably, the NT-proBNP variants have immunological properties (i.e. epitope composition) comparable to those of NT-proBNP. Thus, the variants shall be recognizable by the aforementioned means or ligands used for determination of the amount of the natriuretic peptides. Biological and/or immunological NT-proBNP properties can be detected by the assay described in Karl et al. (Karl 1999, Scand J Clin Invest 59:177-181), Yeo et al. (Yeo 2003, Clinica Chimica Acta 338:107-115). Variants also include posttranslationally modified peptides such as glycosylated or myristylated peptides. Further, a variant in accordance with the present invention is also a peptide or polypeptide which has been modified after collection of the sample, for example by covalent or non-covalent attachment of a label, particularly a radioactive or fluorescent label, to the peptide.

Determining the amount of a peptide or polypeptide may, preferably, comprises the steps of (a) contacting the peptide with a specific detection agent, (b) (optionally) removing non-bound detection agent, (c) measuring the amount of bound detection agent. The bound detection agent will generate an intensity signal. Binding according to the present invention includes both covalent and non-covalent binding. A detection agent according to the present invention can be any compound, e.g., a peptide, polypeptide, nucleic acid, or small molecule, binding to the peptide or polypeptide described herein. Preferred detection agents include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such detection agents are well-known in the art. For example, identification and production of suitable antibodies or aptamers is also offered by commercial suppliers. The person skilled in the art is familiar with methods to develop derivatives of such detection agents with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the detection agent or agent binds specifically to the peptide or polypeptide. Specific binding according to the present invention means that the detection agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the detection agent can be measured by any method known in the art. Preferably, said method is semi-quantitative or quantitative. Further suitable techniques for the determination of a polypeptide or peptide are described in the following.

First, binding of a detection agent may be measured directly, e.g. by NMR or surface plasmon resonance. Second, if the detection agent also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the detection agent may exhibit enzymatic properties itself and the "detection agent/peptide or polypeptide" complex or the detection agent which was bound by the peptide or polypeptide, respectively, may be contacted with a suitable substrate allowing detection by the generation of an intensity signal. For measurement of enzymatic reaction products, preferably the amount of substrate is saturating. The substrate may also be labelled with a detectable label prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured. Third, the detection agent may be coupled covalently or non-covalently to a label allowing detection and measurement of the detection agent. Labelling may be done by direct or indirect methods. The detection agent or substrate may also be "tagged" with one or more tags as known in the art. Such tags may then be targets for higher order detection agents. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immune assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described above.

The amount of a peptide or polypeptide may be, also preferably, determined as follows: (a) contacting a solid support comprising a detection agent for the peptide or polypeptide as specified above with a sample comprising the peptide or polypeptide and (b) measuring the amount peptide or polypeptide which is bound to the support. The detection agent, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The detection agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said detection agent are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labelled, carrying different detection agents. Methods of producing such arrays, for example based on solid-phase chemistry and photo-labile protective groups, are generally known (US 5,744,305).

The term "comparing" as used herein encompasses comparing the amount of the peptide or polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provides the desired assessment in a suitable output format, i.e. the diagnostic result. The said diagnostic result may, preferably, serve as an aid for establishing the final clinical diagnosis by, e.g., a medical practitioner.

Based on the comparison of the amount determined in step a) and the reference amount, it shall be possible to assess whether the cardiovascular system of the pregnant subject has adapted to pregnancy, or not. Therefore, the reference amount is to be chosen so that either a difference or an identity in the compared amounts allows identifying those test subjects which belong into the group of subjects whose cardiovascular system has adapted to pregnancy, or not. The method allows either excluding (rule-out) or identifying (rule-in) a subject as a subject whose cardiovascular system has adapted to pregnancy, or not. Differences in the amounts, i.e. increases or decreases, as used herein, preferably, are differences which are statistically significant. Whether a difference is statistically significant can be determined by the statistical techniques referred to elsewhere herein. Similarly, an identity in the amounts encompasses identical amounts and those differences in the amounts which are not statistically significant and which are within the standard deviations for a measured parameter.

The term "reference amount" as used herein in the context of the aforementioned method refers to an amount which allows for allocation of a subject into either (i) the group of subjects whose cardiovascular system has adapted to pregnancy or (ii) a group of subjects whose cardiovascular system has not adapted to pregnancy. The reference amount applicable for an individual subject may vary depending on various physiological parameters such as age, or subpopulation, as well as on the means used for the determination of the polypeptide or peptide referred to herein. A suitable reference amount may be determined from a reference sample to be analyzed together, i.e. simultaneously or subsequently, with the test sample.

Reference amounts can, in principle, be calculated for a cohort of subjects as specified above based on the average or mean values for a given biomarker by applying standard methods of statistics. In particular, accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity versus specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the y-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the aforementioned method of the present invention, i.e. a threshold which allows to discriminate between subjects whose cardiovascular system has adapted to pregnancy or those whose cardiovascular system has adapted to pregnancy can be generated, preferably, by establishing a ROC for said cohort as described above and deriving a threshold amount therefrom. Dependent on a desired sensitivity and specificity for a diagnostic method, the ROC plot allows deriving suitable thresholds.

Preferably, said reference amounts are derived from a pregnant subject or a group thereof whose cardiovascular system has not adapted to pregnancy or, more preferably, from a pregnant subject or a group whose cardiovascular system has adapted to pregnancy.

Preferably, said reference amount is derived from a pregnant subject or group thereof being at the same stage of gestation as the subject to be tested. More preferably, said reference amounts are derived from a pregnant subject (or group thereof) being in same trimester, or most preferably, being the same week of gestation as the subject to be tested. Therefore, it is particularly envisaged that the reference amount is derived from a pregnant subject or group thereof being in any one of week 1 to 22 of gestation. Even more preferably, the reference amount is derived from a pregnant subject or group thereof being in any one of week 1 to 17 of gestation, or being in any one of week 8 to 17 of gestation. Even more preferably, the reference amount is derived from a pregnant subject or group thereof being in any one of week 14 to 17 of gestation.

Preferably, the following applies as a diagnostic algorithm:
i) an amount of the brain natriuretic peptide in the sample of the pregnant subject to be tested larger than the reference amount or being essentially identical to the reference amount indicates that the cardiovascular system has not adapted to pregnancy, if the reference amount is derived from a pregnant subject or group thereof whose cardiovascular system has not adapted to pregnancy, and/or
ii) an amount of the brain natriuretic peptide in the sample of the pregnant subject to be tested lower than the reference amount or being essentially identical to the reference amount indicates that the cardiovascular system has adapted to pregnancy, if the reference amount is derived from a pregnant subject or group thereof whose cardiovascular system has adapted to pregnancy.

Advantageously, it has been found in the context of the present invention that brain natriuretic peptides are reliable markers for assessing whether the cardiovascular system of a pregnant subject being in any one of week 1 to 22 of gestation has adapted to pregnancy or not. Interestingly, however, brain natriuretic peptides levels are generally increased in pregnant women after conception as compared to non-pregnant subjects. Therefore, the reference amount for carrying out the assessing cardiac adaptation in early pregnancy has to be derived from a pregnant subject, rather than from a non-pregnant subject. The use of reference amounts derived from a non-pregnant subject would lead to a large number of false positive results.

In a preferred embodiment of the method of the present invention, said method further comprises the step of recommending a therapeutic measure if the cardiovascular system of the subject has not adapted to pregnancy.

The term "recommending" as used herein means establishing a proposal for a therapy which could be applied to the subject. However, it is to be understood that applying the actual therapy whatsoever is not comprised by the term. The therapy to be recommended depends on the outcome of the diagnosis provided by the method of the present invention. The recommendation step referred to above can also, preferably, be automated. Preferably, the diagnosis or aid for diagnosis obtained from the step b) of the method of the present invention, i.e. the diagnostic result of the method, will be used to search a database comprising recommendations of therapeutic measures for the individual possible diagnostic results.

Preferably, said therapeutic measure is selected from the group consisting of beta blockers, calcium channel blockers, cardiac glycosides (in particular digoxin), diuretics, in particular loop diuretics and thiazide diuretics and potassium-sparing diuretics, angiotensin-converting enzyme inhibitors and angiotensin receptor-blockers.

Moreover, if the cardiovascular system of the subject has not adapted to pregnancy, reduction of physical exercise is, preferably, recommended.

Also, if the cardiovascular system of the subject has not adapted to pregnancy, the subject shall be, preferably further monitored, in particular by electrocardiography, echocardiography, and/or angiography. Accordingly, the method of the present invention allows to decide whether a subject shall be further monitored or not.

It is to be understood that the definitions and explanations of the terms made above and below apply accordingly for all embodiments described in this specification and the accompanying claims (except stated otherwise).

Moreover, the present invention relates to a method for assessing in a pregnant subject whether fluid retention is the cause of abnormal weight gain during pregnancy, comprising the steps of
a. determining the amount of a brain natriuretic peptide in a sample from a pregnant subject,
b. comparing the, thus, determined amount of said brain natriuretic peptide to a reference amount, whereby it is to be assessed whether fluid retention is the underlying cause of abnormal weight gain during pregnancy.

Preferably, it is assessed whether fluid retention is the cause of abnormal weight gain during pregnancy, or not, by carrying out the further step of c) assessing whether fluid retention is the cause of abnormal weight gain during pregnancy, or not, based on the result of the comparison carried out in step b).

The term "subject" as used in accordance with the aforementioned method relates to animals, preferably mammals, and, more preferably, humans. The subject referred to herein is a pregnant subject and, thus, preferably, a female subject. Moreover, the subject shall be pregnant, and, thus, shall be female. In accordance with the aforementioned method, the subject, preferably, shall be in the second trimester, or, more preferably, in the third trimester of pregnancy. The second trimester of pregnancy encompasses weeks 13 to 26 of pregnancy, the third trimester weeks 27 to 42 (up to the birth). The duration of pregnancy is, preferably, calculated from the date of conception.

Also preferably, said subject shall not exhibit impaired renal function. Moreover, the subject in the context of the method of the present invention preferably is apparently healthy with respect to a cardiovascular disease (for an explanation of this term see elsewhere). Also preferably, the cardiovascular system of the subject shall have adapted to pregnancy (see also above).

The subject to be tested in accordance with the aforementioned method shall show abnormal weight gain during pregnancy. The expression "abnormal weight gain" is understood by the skilled person. The term "abnormal weight gain", as used herein preferably, refers to a weight gain that is larger than normal weight gain in a control group, in particular, larger than the median weight gain in a control group. More preferably, the term refers to a weight gain that is more than 10%, or, more preferably, more than 20% larger than the normal weight gain, in particular the median weight gain, in a control group. Preferably, the subjects of the control group shall be in the same stage of pregnancy, in particular in the same trimester or week of gestation (see also below). Preferably, the weight gain is calculated for the period of one week.

It is known that the weight gain during pregnancy may depend on the pre-pregnancy body mass index. Therefore, the control group preferably consists of pregnant subjects having the same or essentially the same pre-pregnancy body mass index. For example, if the subject to be tested has a pre-pregnancy body mass index of lower than 18.5 kg/m², the control group preferably shall consist of subjects having also a pre-pregnancy body mass index of lower than 18.5 kg/m². If the subject to be tested has a pre-pregnancy body mass index of equal or larger than 18.5 but lower than 25 kg/m², the control group preferably shall consist of subjects having a pre-pregnancy body mass index (BMI) within this range. If the subject to be tested has a pre-pregnancy body mass index of equal or larger than 25 but lower than 30 kg/m², the control group preferably shall consist of subjects having a pre-pregnancy body mass index within this range. If the subject to be tested has a pre-pregnancy body mass index of equal or larger than 30 kg/m², the control group preferably shall consist of subjects having of equal or larger than 30 kg/m². The following overview lists median weight increases for various BMIs. The indicated weight increases are, thus, considered as normal weight increase during the second and third trimester of pregnancy:

| | |
|---|---|
| Pre-pregnancy BMI | Median weight gain (kg/week) during second and third trimester |
| lower than 18.5 kg/m² | 0.5 kg/week |
| equal or larger than 18.5 but lower than 25 kg/m² | 0.4 kg/week |
| equal or larger than 25 but lower than 30 kg/m² | 0.3 kg/week |
| equal or larger than 30 kg/m² | 0.2 kg/week |

Preferably, a weight gain during the second or third trimester larger than the aforementioned median weight gains is considered as abnormal weight gain (with respect to the BMI). More preferably, a weight gain of more than 10%, or, even more preferably, of more than 20% larger than the aforementioned weight gains is considered as abnormal weight gain (with respect to the pre-pregnancy BMI). Most preferably, a weight gain of more than 600 g/week during at least one week of the second or third trimester is considered as an abnormal weight gain. The "at least one week", preferably, refers to one week, two weeks, three weeks, or more.

Further preferred weight gains during the second or third trimester of pregnancy that are considered as abnormal are listed herein below (with respect to the pre-pregnancy body mass index (BMI))

| | |
|---|---|
| Pre-pregnancy BMI | Abnormal weight gain |
| lower than 18.5 kg/m² | larger than 0.6 kg/week, or more preferably, larger than 0.7 kg/week |
| equal or larger than 18.5 but lower than 25 kg/m² | larger than 0.5 kg/week, or more preferably, larger than 0.6 kg/week |
| equal or larger than 25 but lower than 30 kg/m² | larger than 0.4 kg/week, or more preferably, larger than 0.5 kg/week |
| equal or larger than 30 kg/m² | larger than 0.3 kg/week, or more preferably, larger than 0.4 kg/week |

In the context of the method of the present invention, it shall be assessed whether abnormal weight gain during pregnancy is caused by fluid retention. The term "fluid retention", as used herein, preferably, refers to i) intravascular fluid retention, or ii) intravascular fluid retention accompanied by extravascular fluid retention. The term "intravascular fluid retention", preferably, refers to the accumulation of fluid, in particular of water, in the circulatory system, whereas the term "extravascular fluid retention", preferably, refers to the accumulation of fluid, in particular of water, within the tissues and/or cavities of the body. It is to be understood that the fluid retention, and, thus, the accumulation of fluid in the circulatory system, or within the tissues and/or cavities of the body shall be abnormal, i.e. above normal. The term abnormal in conjunction with fluid retention during pregnancy is understood by the skilled person. In particular, a fluid retention is considered as abnormal, if the amount of fluid that is accumulated is, increasing order of preference, at least 20%, 10% or 5% larger than the mean accumulation of fluid in a control group. The control group, preferably, shall consist of subjects being in the same stage of pregnancy as the subject to be tested, in particular in the same week or trimester of pregnancy. Preferably, said subjects have the same or essentially the same pre-pregnancy BMI.

The reference amount in the context of the aforementioned method is, preferably, derived from a pregnant subject (or group thereof) being in the same stage of gestation, in particular the reference amount shall be derived from a subject (or from a group thereof) being in the same week or same trimester of gestation.

Moreover, it is particularly preferred that the reference amount is derived from a pregnant subject (or group thereof) with abnormal weight gain during pregnancy, in particular the second and/or third trimester (the term "abnormal weight gain" is explained elsewhere herein, the definition also applies accordingly). Said subject with abnormal weight gain during pregnancy shall be either i) known to have fluid retention as the cause of abnormal weight gain, or ii) shall be known not to have fluid retention as the cause of abnormal weight gain. The weight gain of the subject as set forth in ii), preferably, has causes other than fluid retention, e.g. an excess calorie intake.

The following, preferably, applies as diagnostic algorithm:
i) an amount of the brain natriuretic peptide, in particular of NT-proBNP, being essentially identical or larger than the reference amount indicates that fluid retention is the cause of abnormal weight gain, if the reference amount is derived from a pregnant subject known to have fluid retention as the cause of abnormal weight gain during pregnancy,
   and/or
ii) an amount of the brain natriuretic peptide, in particular of NT-proBNP, being essentially identical or lower than the reference amount indicates that fluid retention is not the cause of abnormal weight gain, if the reference amount is derived from a pregnant subject known not to have fluid retention as the cause of abnormal weight gain during pregnancy

Surprisingly, it was shown in the context of the method of the present invention that brain natriuretic peptides are reliable markers for assessing the cause of abnormal weight gain during pregancy. In particular, it was shown that increased levels of a brain natriuretic peptides in subjects with abnormal weight gain indicates that abnormal weight gain is caused by abnormal fluid retention. The present invention is advantageous since it allows for easily assessing whether abnormal weight gain during pregnancy is caused by fluid retention or not.

In a preferred embodiment of the method of the present invention, said method further comprises the step of recommending a therapeutic measure if it has been found fluid retention is the cause of abnormal weight gain.

The term "recommending" as used herein means establishing a proposal for a therapy which could be applied to the subject. However, it is to be understood that applying the actual therapy whatsoever is not comprised by the term. The therapy to be recommended depends on the outcome of the diagnosis provided by the method of the present invention. The recommendation step referred to above can also, preferably, be automated. Preferably, the diagnosis or aid for diagnosis obtained from the step b) of the method of the present invention, i.e. the diagnostic result of the method, will be used to search a database comprising recommendations of therapeutic measures for the individual possible diagnostic results.

Moreover, the present invention relates to the use of a brain natriuretic peptide or to the use of a detection agent which specifically binds thereto in a sample of a subject being in any one of week 1 to 22 of gestation for assessing cardiac adaptation to pregnancy.

Moreover, the present invention relates to the use of a brain natriuretic peptide or to the use of a detection agent which specifically binds thereto in a sample of a pregnant subject with abnormal weight gain during pregnancy for assessing whether fluid retention is the cause of said abnormal weight gain.

The term "detection agent" as used herein refers to an agent which is capable of specifically recognizing and binding to a brain natriuretic peptide present in a sample. The agent shall allow for direct or indirect detection of the complex formed by the said agent and the biomarker. Direct detection can be achieved by including into the agent a detectable label. Indirect labelling may be achieved by a further agent which specifically binds to the complex comprising the biomarker and the detection agent wherein the said further agent is than capable of generating a detectable signal. Suitable compounds which can be used as detection agents are well known in the art. Preferably, the detection agent is an antibody or aptamere which specifically binds to the biomarker protein or a nucleic acid encoding the biomarker. The term "antibody" as used herein includes both polyclonal and monoclonal antibodies, as well as any modifications or fragments thereof, such as Fv, Fab and F(ab) ₂ fragments. The antibody shall be capable of specifically binding to the brain natriuretic peptide.

Moreover, the present invention relates to a device adapted for carrying out the method of the present invention for assessing cardiac adaptation to pregnancy in a pregnant subject being in any one of week 1 to 22 of gestation, comprising
a. an analyzing unit comprising a detection agent which specifically binds to a brain natriuretic peptide, said unit being adapted for determining the amount of a brain natriuretic peptide in a sample of a pregnant subject; and
b. an evaluation unit for comparing the determined amount with a reference amount whereby cardiac adaptation to pregnancy can be diagnosed, said unit comprising a database with a value for a reference amount (or values for reference amounts) for a brain natriuretic peptide and a computer-implemented algorithm for carrying out the comparison step.

Preferred reference amounts are described herein above.

Moreover, the present invention relates to a device adapted for carrying out the method of the present invention for assessing whether fluid retention is the cause of abnormal weight gain during pregnancy, comprising
a. an analyzing unit comprising a detection agent which specifically binds to a brain natriuretic peptide, said unit being adapted for determining the amount of a brain natriuretic peptide in a sample of a pregnant subject; and
b. an evaluation unit for comparing the determined amount with a reference amount whereby it can be assessed whether fluid retention is the cause of abnormal weight gain during pregnancy, said unit comprising a database with a value for a reference amount (or values for reference amounts) for a brain natriuretic peptide and a computer-implemented algorithm for carrying out the comparison step.

Preferred reference amounts are described herein above.

The term "device" as used herein relates to a system comprising the aforementioned units operatively linked to each other as to allow the diagnosis or assessment according to the methods of the invention. Preferred detection agents which can be used for the analyzing unit are disclosed elsewhere herein. The analyzing unit, preferably, comprises said detection agents in immobilized form on a solid support which is to be contacted to the sample comprising the biomarkers the amount of which is to be determined. Moreover, the analyzing unit can also comprise a detector which determines the amount of detection agent which is specifically bound to the biomarker(s). The determined amount can be transmitted to the evaluation unit. Said evaluation unit comprises a data processing element, such as a computer, with an implemented algorithm for carrying out a comparison between the determined amount and a suitable reference (e.g. a reference amount, or the amount of the marker in a first or second sample from the subject). Suitable references can be derived from samples of subjects to be used for the generation of reference amounts as described elsewhere herein above. The results may be given as output of parametric diagnostic raw data, preferably, as absolute or relative amounts. It is to be understood that these data will need interpretation by the clinician. However, also envisaged are expert system devices wherein the output comprises processed diagnostic raw data the interpretation of which does not require a specialized clinician.

Moreover, the present invention envisages a kit adapted for carrying out the method of the present invention for assessing cardiac adaptation to pregnancy in a subject being in any one of week 1 to 22 of pregnancy, said kit comprising a detection agent for a brain natriuretic peptide. Moreover, the kit further may comprise instructions for carrying out the said method.

Moreover, the present invention envisages a kit adapted for carrying out the method of the present invention for whether fluid retention is the cause of abnormal weight gain during pregnancy, said kit comprising a detection agent for a brain natriuretic peptide. Moreover, the kit further may comprise instructions for carrying out the said method.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as an optical storage medium (e.g., a Compact Disc) or directly on a computer or data processing device. Moreover, the kit may, preferably, comprise standards for reference amounts as described elsewhere herein in detail.

All references cited in this specification are herewith incorporated by reference with respect to their entire disclosure content and the disclosure content specifically mentioned in this specification.

### EXAMPLES

**Example 1:** Determination of NT-proBNP

NT-proBNP was determined with sandwich immuno-assays comprising two monoclonal antibodies specific for the respective peptide. The first of these iv biotinylated and the second one in labelled with a Tris(2,2'-bibyridyl)ruthemium (II)-complex. In a first incubation step both antibodies are incubated with the sample. A sandwich complex comprising the peptide to be determined and the two different antibodies is formed. In a next incubation step streptavidin-coated beads are added to this complex. The beads bind the sandwich complexes. The reaction mixture is then aspirated into a measuring cell where the beads are magnetically captured on the surface of the electrode. The application of a voltage then induces a chemiluminescent emission from the ruthenium complex which is measured by a photomultiplier. The emitted amount of light is dependent on the amount of sandwich complexes on the electrode. NT-proBNP amounts between 2 pg/ml and 35,000 pg/ml can be measured.

### Example 2: Patient cohorts

Pregnant women: A total of 86 pregnant women, median age 29 years without previous underlying disease including heart disease and hypertension were included into the study, all pregnant women had a clinically uneventful course during pregnancy and thus were considered healthy. In addition 24 pregnant women, mean age 31 years, who developed a gestational diabetes mellitus, were also included into the study. Pregnant women with gestational diabetes mellitus had no significant pre-existing disorder and did not develop complications during pregnancy except gestational diabetes mellitus. Kidney function was normal in all subjects as assessed by normal creatinine levels in serum. All pregnant women were followed clinically according to standard procedures including weekly recording of body weight. Most pregnant women could be followed throughout pregnancy and until 24 weeks after delivery. Timepoints of analysis were grouped into 8 - 13 weeks, 14 - 17 weeks, 18 - 22 weeks, 23 - 27 weeks, 28 to 32 weeks, 33 to 37 weeks, 38 weeks to delivery, 1 week post delivery, 6 weeks post delivery and 24 weeks post delivery. Non-pregnant controls: NT-proBNP was determined in 213 healthy females between 25 and 35 years old (median 29 years). The subjects belong to a group apparently healthy blood donors and to a group of individuals from general practitioners without cardiac disease (total number of subjects in the study: N=2264).

### Example 3: Natriuretic peptides are generally increased in the first weeks of pregnancy

Results of "healthy pregnant women" were grouped as described an median as well as 25^{th} and 75^{th} percentile values are given.

### "Healthy pregnant women"

| NT-pro BNP (week of gestation) | pg/ml |
|---|---|
| 8 - 13 | 67 (48 - 119) |
| 14-17 | 68 (47-87) |
| 18-22 | 53 (39-93) |
| 23 - 27 | 44 (29 - 61) |
| 28 - 32 | 36 (23-56) |
| 33-37 | 42 (26-66) |
| 38 - delivery | 43 (28 - 67) |
| 1 week pp | 95 (66 - 184) |
| 6 weeks pp | 77 (34-94) |
| 24 weeks pp | 63 (34-80) |

Median levels up to 67 pg/ml were observed in the early stage of pregnancy. In the control group (213 non-pregnant females without cardiac disease, between 25 and 35 years old (median 29 years)) the median was 38 pg/ml (25^{th} percentile: 21 pg/ml; 75^{th} percentile: 60 pg/ml). Thus, the level of NT-proBNP in pregnant women in the early phase of pregnancy is increased as compared to the level of NT-proBNP in non-pregnant women.

The data in the table illustrate, that in healthy pregnant women the heart apparently adapts to the pregnancy within the first 20 weeks indicated by median decreasing NT-pro BNP levels. A further adaptation phase occurs after delivery with significantly increased NT-pro BNP levels 1 week pp, 24 weeks pp NT-pro BNP levels have returned to levels in early pregnancy.

A more detailed analysis of the early adaptation phase indicates that 11/86 subjects of apparently healthy pregnant women fail to adapt appropriately. This is shown in examples

The data in the table illustrate, that in healthy pregnant women the heart apparently adapts to the pregnancy within the first 20 weeks indicated by median decreasing NT-pro BNP levels. A further adaptation phase occurs after delivery with significantly increased NT-pro BNP levels 1 week pp, 24 weeks pp NT-pro BNP levels have returned to levels in early pregnancy.

In the majority of pregnant women analyzed in this study, NT-proBNP levels were not increased, e.g. in case 40, 72, 211 and 217.

| 8-13 | 14-17 | 18-22 | week |
|---|---|---|---|
| Case 40 | 46 | 29 | 39 |
| Case 72 | 67 | 35 | 45 |
| Case 211 | 51 | 45 | 16 |
| Case 217 | 55 | 46 | 27 |

While the above cases represent examples with variation of adaptation within normal the following cases represent abnormal adaptation indicated by significantly increased levels of NT-proBNP during weeks 8 to 22 of pregnancy. After week 22, the levels of NT-proBNP dropped in cases 13, 94, 176, and 232.

| Week | 8-13 | 14-17 | 18-22 |
|---|---|---|---|
| Case 232 | 272 | 251 | 198 |
| Case 176 | 196 | 179 | 95 |
| Case 94 | 113 | 119 | 149 |
| Case 13 | 200 | 190 | 194 |
| Case 35 | 270 | 333 | 109 |
| | | | |

| Week | 23-27 | 28-32 | 33-37 |
|---|---|---|---|
| Case 232 | 73 | 75 | 76 |
| Case 176 | 58 | | 78 |
| Case 94 | 41 | 36 | 26 |
| Case 13 | 63 | 85 | 101 |
| Case 35 | 143 | 104 | 111 |

As can be seen from these cases there is an abnormal adaptation to the pregnancy situation in these women, this is supported by the fact, that among 6 women who hat NT-pro BNP above the 75 th percentile of normal 5 were poor early adapters indicating poor early adaptation in pregnancy as an occult sign of abnormal cardiac function.

### Example 4:

Another finding of the study was that in some patients short term increases of NT-proBNP were observed at single timepoints. Interestingly, this predominantly occurred in patients at later stages of pregnancy. These patients usually did not have increased NT-proBNP levels in the early stage of pregnancy. Examples are shown below:

| | **14-17** | 18-22 | **23-27** | 28-32 | **33-37** | 38-d |
|---|---|---|---|---|---|---|
| **Healthy** | | | | | | |
| Case 95 | | 73 | 118 | 55 | | |
| Case 215 | | | 45 | 74 | 33 | |
| Case 187 | | | 57 | 102 | 65 | |

| **Gestational Diabetes** | | | | | | |
|---|---|---|---|---|---|---|
| Case 20 | | | 37 | 113 | 46 | |
| Case 101 | | | | 15 | 98 | |
| Case 133 | | | | | 832 | 97 |
| Case 224 | | | | | 52 | 108 |

Interestingly, 12 out of 18 patients with an increase of NT-proBNP at a later stage of pregnancy showed abnormal weight gain. The data suggest that NT-proBNP can be used as marker for assessing whether fluid retention is the cause of abnormal weight gain. Only a few patients had abnormal weight gain without having increased levels of NT-proBNP. This indicates that fluid retention is not the cause of weight gain in these patients.

### Example 5

A 31 year old pregnant woman at gestational week 18 has a NT-pro BNP of 129 pg/ml, she reports no symptoms and has no history of a cardiac disease including hypertension. Her blood pressure at the time of examination is normal. The pregnancy runs an uneventful course, 24 weeks after delivery she has another NT-pro BNP test which is 92 pg/ml. An echocardiography reveals diastolic dysfunction. She is informed that the elevated NT-pro BNP early in pregnancy was an early indicator of inadequate response to stress of the heart and that she needs further assessment of her heart and follow up.

### Example 6

A 34 year old pregnant women has a NT-pro BNP of 89 pg/ml measured in gestational week 17. She has no history of a cardiac disease or hypertension and no apparent risk factors for a cardiac disease. Despite the increased levels of NT-proBNP, she has no symptoms of maladaption to pregnancy. The pregnancy is uneventful and she delivers a healthy child. 24 weeks after delivery her NT-pro BNP is 62 pg/ml and the echocardiography reveals no abnormalities.

### Example 7

A 29 years old pregnant women without obvious cardiac disease in history is in her 34 th gestational week, she monitors her weight carefully and has recognised a weight increase of 650 g in the past week, she has an NT-pro BNP of 98 pg/ml with NT-pro BNP values within normal 1 month ago (i.e. lower). She is diagnosed with fluid retention and is advised to restrict salt consumption and to return for follow up in one month or if weight increase continues to be above normal in the following two weeks.

### Example 8

A 32 years old pregnant woman in her 36th gestational week has gained weight in the past 2 weeks well above normal 500 g/ week. She reports a good appetite but she is afraid that she has retained abnormal fluid. Her NT-pro BNP is 38 pg/ml well within normal with respect to gestational week. She is informed that there is no evidence of cardiac dysfunction and that weight gain is associated with increased calory intake.

### Example 9

A 30 year old female sees her gynaecologist as she and her husband want to have children, she has no history of a cardiac disease and no hypertension but she has smoked heavily for 8 years. Her NT-pro BNP is 71 pg/ml and she has a diastolic dysfunction on echocardiography. She is told that she has evidence of a cardiac disease which may affect adaptation of cardiac function specifically early in pregnancy and to have NT-pro BNP carefully monitored and may take further advice from a cardiologist during pregnancy.

### Example 10

A 24 year old pregnant woman who was previously completely healthy has a NT-pro BNP of 141 pg/ml measured in week 17 of pregnancy. An echocardiography reveals suspicion of an atrial septum defect which has escaped physical examination in the past. She is told that increased NT-pro BNP values would likely be the result of this previously unrecognised inherited septum defect. She is told no to exercise excessively and to follow NT-pro BNP values during pregnancy at regular interval (approximately 4 weeks) and to have a thorough cardiovascular examination, preferably more then 24 weeks after delivery.

### Conclusion

The present study has revealed important results with respect to cardiac patient care in pregnancy where significant changes occur specifically in early and to a lesser extent in late pregnancy, these changes extent well beyond delivery. One important aspect is abnormal adaptation in the first trimester of pregnancy, this abnormal adaptation is associated with cardiac dysfunction after delivery. Another aspect of the present study is the assessment of abnormal weight gain in pregnancy where increased NT-pro BNP values are associated with fluid retention and normal NT-pro BNP values exclude significant fluid retention. This NT-pro BNP adds important diagnostic aspects to the care in pregnancy.

## Claims

1. A method for assessing cardiac adaptation to pregnancy in a pregnant subject being in any one of week 1 to 22 of gestation, said method comprising the steps of
a. determining the amount of a brain natriuretic peptide in a sample from said-pregnant subject, and
b. comparing the, thus, determined amount of said brain natriuretic peptide to a reference amount, said reference amount being derived from a pregnant subject being in the same stage of gestation, whereby cardiac adaptation to pregnancy is to be assessed.

2. The method of claim 1, wherein the subject is in any one of weeks 8 to 17 of pregnancy.

3. The method of claims 1 and 2, wherein said brain natriuretic peptide is NT-proBNP.

4. The method of any one of claims 1 to 3, wherein the sample is a blood, serum or plasma sample.

5. The method of any one of claims 1 to 4, wherein said reference amount is derived from a pregnant subject or group of subjects whose cardiovascular system has adapted to pregnancy or wherein said reference amount is derived from a pregnant subject or group of subjects whose cardiovascular system has adapted to pregnancy.

6. A method for assessing in a pregnant subject being in the second or third trimester of pregnancy whether fluid retention is the cause of abnormal weight gain during pregnancy, comprising the steps of
a. determining the amount of a brain natriuretic peptide in a sample from said-pregnant subject, and
b. comparing the, thus, determined amount of said brain natriuretic peptide to a reference amount, whereby it is to be assessed whether fluid retention is the underlying cause of abnormal weight gain during pregnancy.

7. The method of claim 6, wherein the pregnant subject is in the third trimester of pregnancy.

8. The method of claims 6 and 7, wherein the fluid retention is an accumulation of fluid in the circulatory system.

9. The method of any one of claims 6 to 8, wherein the reference amount is derived from a pregnant subject with abnormal weight gain during pregnancy, said subject being known to have fluid retention as the cause of said abnormal weight gain.

10. The method of claim 9, wherein an amount of said brain natriuretic peptide being essentially identical or larger than the reference amount indicates that fluid retention is the cause of abnormal weight gain.

11. The method of any one of claims 9 to 10, wherein the brain natriuretic peptide is NT-proBNP and/or wherein the sample is blood, serum or plasma.

12. Use of a brain natriuretic peptide or of detection agent which specifically binds thereto for assessing cardiac adaptation to pregnancy in a sample of a pregnant subject being in any one of week 1 to 22 of gestation, or for assessing whether fluid retention is the cause of abnormal weight gain during pregnancy in a sample of a pregnant subject being in the second or third trimester of pregnancy.

13. A device adapted for carrying out the method of the present invention for assessing cardiac adaptation according to any one of claims 1 to 5, comprising
i. an analyzing unit comprising a detection agent which specifically binds to a brain natriuretic peptide, said unit being adapted for determining the amount of a brain natriuretic peptide in a sample of a pregnant subject; and
ii. an evaluation unit for comparing the determined amount with a reference amount whereby cardiac adaptation to pregnancy can be assessed, said unit comprising a database with a value for a reference amount (or values for reference amounts) as defined in claim 1 or 5 and a computer-implemented algorithm for carrying out the comparison step.

14. A device adapted for carrying out the method according to any one of claims 6 to 11, comprising
i. an analyzing unit comprising a detection agent which specifically binds to a brain natriuretic peptide, said unit being adapted for determining the amount of a brain natriuretic peptide in a sample of a pregnant subject; and
ii. an evaluation unit for comparing the determined amount with a reference amount whereby it can be assessed whether fluid retention is the cause of abnormal weight gain during pregnancy, said unit comprising a database with a value for a reference amount (or values for reference amounts) as defined in claim 9 and a computer-implemented algorithm for carrying out the comparison step.
